# EUROPÄISCHE PATENTSCHRIFT

(11) **EP 1 021 419 B1**
(45) Veröffentlichungstag und Bekanntmachung des Hinweises auf die Patenterteilung: **03.12.2003**
(21) Anmeldenummer: 98951472.4
(22) Anmeldetag: 26.09.1998
(51) Int. Cl.: C07D 249/12

(54) **VERFAHREN ZUR HERSTELLUNG VON TRIAZOLINTHION-DERIVATEN**
PROCESS FOR PREPARING TRIAZOLINE THIONE DERIVATIVES
PROCEDE DE PRODUCTION DE DERIVES DE TRIAZOLINETHIONE

(30) Priorität: 10.10.1997 DE 19744706
(43) Veröffentlichungstag der Anmeldung: 26.07.2000
(73) Patentinhaber: Bayer CropScience AG, 40789 Monheim (DE)
(72) Erfinder: JAUTELAT, Manfred, D-51399 Burscheid (DE); ERDMAN, David, D-51061 Köln (DE)
(86) Internationale Anmeldenummer: EP9806127
(87) Internationale Veröffentlichungsnummer: WO99019307

(56) Entgegenhaltungen:
- WO-A-96/16048
- DE-A- 19 620 590

## Beschreibung

Die vorliegende Erfindung betrifft ein neues Verfahren zur Herstellung von Triazolinthion-Derivaten, die als Wirkstoffe mit mikrobiziden, insbesondere fungiziden Eigenschaften bekannt sind.

Es ist bereits bekannt, daß sich Triazolinthion-Derivate herstellen lassen, indem man die entsprechenden Triazol-Derivate nacheinander mit starken Basen und Schwefel umsetzt und dann hydrolysiert (vgl. WO-A 96-16 048). Die Ausbeuten bei diesem Verfahren sind gut. Nachteilig ist aber, daß Umsetzungsbedingungen erforderlich sind, die im technischen Maßstab nur schwierig einzuhalten sind. So ist zum Beispiel ein Arbeiten mit Butyl-lithium als Base nicht nur sehr kostspielig, sondern auch sicherheitstechnisch äußerst aufwendig.

Weiterhin ist bekannt, daß man Triazolinthion-Derivate erhält, wenn man die entsprechenden Triazol-Derivate direkt mit Schwefel bei hohen Temperaturen umsetzt (vgl. WO-A 96-16 048). Ungünstig an dieser Methode ist jedoch, daß die gewünschten Produkte nur in relativ niedrigen Ausbeuten anfallen.

Es wurde nun gefunden, daß sich Triazolinthion-Derivate der Formel 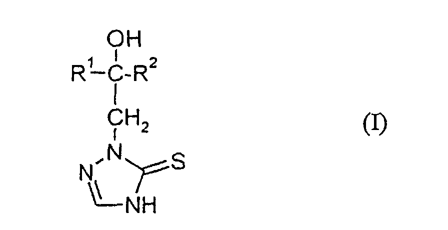 in welcher
- R¹: für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
- R²: für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
durch Reaktion von Triazol - Derivaten der Formel 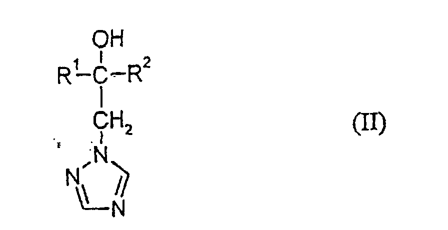 in welcher
R¹ und R² die oben angegebenen Bedeutungen haben,
mit Schwefel in Gegenwart eines aprotischen, polaren Verdünnungsmittels, dadurch herstellen lassen, daß man die Umsetzung bei Temperaturen zwischen 150° C und 160° C durchführt,
wobei die Mengen an den Reaktionskomponenten so gewählt werden, daß auf 1 Mol an Triazol-Derivat der Formel (II) zwischen 8 und 13 Mol Schwefel vorhanden sind,
und wobei während der Umsetzung Luft über das Reaktionsgemisch geleitet wird.

Es ist als ausgesprochen überraschend zu bezeichnen, daß sich Triazolinthion-Derivate der Formel (I) nach dem erfindungsgemäßen Verfahren in wesentlich höheren Ausbeuten oder unter erheblich einfacheren Bedingungen herstellen lassen als nach den bisher bekannten Methoden.

Das erfindungsgemäße Verfahren zeichnet sich durch eine Reihe von Vorteilen aus. So ermöglicht es, wie schon erwähnt, die Synthese von Triazolinthion-Derivaten der Formel (I) in hoher Ausbeute. Günstig ist auch, daß die benötigten Ausgangsstoffe und Reaktionskomponenten einfach herstellbar und auch in größeren Mengen verfügbar sind. Ein weiterer Vorteil besteht schließlich darin, daß die Durchführung der einzelnen Reaktionsschritte und die Isolierung der Reaktionsprodukte keinerlei Schwierigkeiten bereitet.

Verwendet man 2-(1-Chlor-cyclopropyl)-1-(2-chlor-phenyl)-3-(1,2,4-triazol-1-yl)-propan-2-ol als Ausgangssubstanz und setzt diese mit der zehnfach molaren Menge an Schwefel-Pulver in Gegenwart von Dimethylformamid bei 150°C um, so kann der Verlauf des erfindungsgemäßen Verfahrens durch das folgende Formelschema veranschaulicht werden.

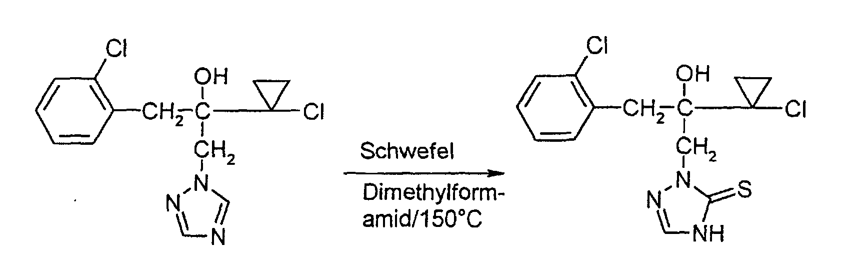

Die bei der Durchführung des erfindungsgemäßen Verfahrens als Ausgangsstoffe benötigten Triazol-Derivate sind durch die Formel (II) allgemein definiert. Bevorzugt einsetzbar sind Verbindungen der Formel (II), in denen
- R¹: für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
- R²: für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-cyclopropyl, Cyclopropyl, 1-Methyl-cyclopentyl oder 1-Ethyl-cyclopentyl steht.

Die Triazol-Derivate der Formel (II) sind bekannt oder lassen sich nach bekannten Methoden herstellen (vgl. EP-A 0 015 756, EP-A 0 040 345, EP-A 0 052 424, EP-A 0 061 835, EP-A-0 297 345 und EP-A 0 470 463).

Schwefel wird bei der Durchführung des erfindungsgemäßen Verfahrens im allgemeinen in Form von Pulver eingesetzt.

Als Verdünnungsmittel kommen bei der Durchführung des erfindungsgemäßen Verfahrens alle üblichen aprotischen, polaren organischen Solventien in Betracht. Vorzugsweise verwendbar sind Amide, wie Dimethylformamid, ferner N-Alkyl-pyrrolidone, wie N-Octyl-pyrrolidon und N-Dodecyl-pyrrolidon, und außerdem N-Alkylcaprolactame, wie N-Methyl-caprolactam und N-Octyl-caprolactam.

Bei der Durchführung des erfindungsgemäßen Verfahrens arbeitet man im allgemeinen unter Atmosphärendruck. Es ist aber auch möglich, unter erhöhtem Druck zu arbeiten.

Bei der Durchführung des erfindungsgemäßen Verfahrens wird während der Umsetzung ein Luftstrom über das Reaktionsgemisch geleitet. Die Aufarbeitung erfolgt nach üblichen Methoden. Im allgemeinen geht man in der Weise vor, daß man das Reaktionsgemisch filtriert, dann unter vermindertem Druck einengt, den verbleibenden Rückstand in einem mit Wasser wenig mischbaren organischen Solvens aufnimmt, die organische Phase wäscht, trocknet und einengt. Das dabei erhaltene Produkt kann nach üblichen Methoden, zum Beispiel durch Chromatographie oder Umkristallisation, von eventuell vorhandenen Verunreinigungen befreit werden.

Die erfindungsgemäß herstellbaren Triazolinthion-Derivate können in der "Thiono"-Form der Formel 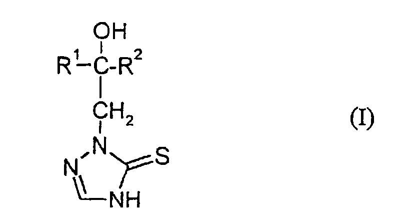 oder in der tautomeren "Mercapto"-Form der Formel 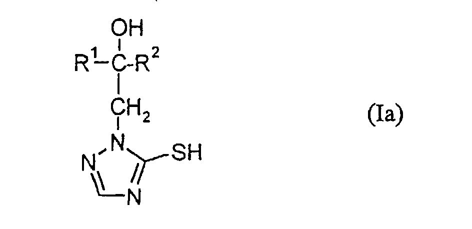 vorliegen.

Der Einfachheit halben wird jeweils nur die "Thiono"-Form aufgeführt.

Die erfindungsgemäß herstellbaren Triazolinthion-Derivate sind als Wirkstoffe mit mikrobiziden, insbesondere fungiziden Eigenschaften bekannt (vgl. WO-A 96-16 048).

Die Durchführung des erfindungsgemäßen Verfahrens wird durch die folgenden Beispiele veranschaulicht.

### Herstellungsbeispiele

### Beispiel 1

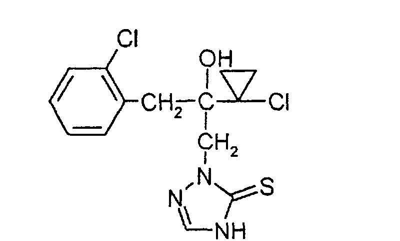

Ein Gemisch aus 50 g (0,16 Mol) 2-(1-Chlor-cyclopropyl)-1-(2-chlor-phenyl)-3-(1,2,4-triazol-1-yl)-propan-2-ol, 50 g (1,6 Mol) Schwefel-Pulver und 100 ml Dimethylformamid wird 16 Stunden unter Rühren auf 150°C erhitzt. Dabei wird ein kontinuierlicher Luftstrom über das Reaktionsgemisch geleitet. Anschließend wird das Reaktionsgemisch auf Raumtemperatur abgekühlt und filtriert. Der Filterrückstand wird mit 100 ml Dimethylformamid nachgewaschen. Die vereinigten organischen Phasen werden unter vermindertem Druck eingeengt, und der verbleibende Rückstand wird dreimal mit je 100 ml auf 60°C erwärmten Toluol extrahiert. Die vereinigten organischen Phasen werden zunächst mit 100 ml 10 %-iger wäßriger Salzsäure und dann dreimal mit je 100 ml 10 %-iger wäßriger Natronlauge gewaschen. Die vereinigten, wäßrig alkalischen Phasen werden noch einmal mit Toluol extrahiert. Die vereinigten organischen Phasen werden über Natriumsulfat getrocknet und dann unter vermindertem Druck eingeengt. Man erhält dabei 6,8 g eines Produktes, das gemäß HPLC-Analyse zu 83,5 % aus 2-(1-Chlor-cyclopropyl)-1-(2-chlor-phenyl)-3-(1,2,4-triazol-1-yl)-propan-2-ol besteht.

Die wäßrig alkalische Phase wird durch Zugabe von 50 ml konzentrierter Salzsäure angesäuert und dann dreimal mit jeweils 100 ml warmem Toluol extrahiert. Die vereinigten organischen Phasen werden über Natriumsulfat getrocknet und danach unter vermindertem Druck bis auf ein Volumen von etwa 40 ml eingeengt. Nach dem Abkühlen und Animpfen kristallisiert ein gelber Feststoff aus, der abfiltriert wird. Man erhält auf diese Weise 41,2 g eines Produktes, das gemäß HPLC-Analyse zu 91,4 % aus 2-(1-Chlor-cyclopropyl)-1-(2-chlor-phenyl)-3-(4,5-dihydro-1,2,4-triazol-5-thiono-1-yl)-propan-2-ol besteht. Die Ausbeute errechnet sich danach zu 75 % der Theorie.

### Vergleichsbeispiele

### Beispiel A

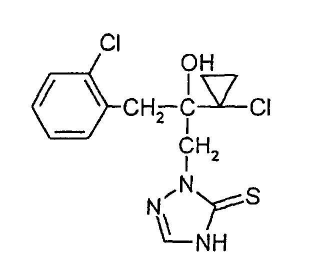

Ein Gemisch aus 3,12 g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g eines Produktes, das gemäß gaschromatografischer Analyse zu 95 % aus 2-(1-Chlor-cyclopropyl)-1-(2-chlorphenyl)-3-(4,5-dihydro-1,2,4-triazol-5-thiono-1-yl)-propan-2-ol besteht. Nach Umkristallisation aus Toluol erhält man diese Substanz als Feststoff, der bei 138 bis 139°C schmilzt.

### Beispiel B

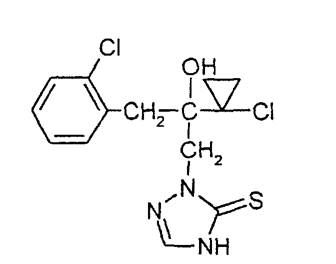

Ein Gemisch aus 3,12 g (10 mmol) 2-(1-Chlor-cyclopropyl)-1-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cyclopropyl)-1-(2-chlorphenyl)-3-(4,5-dihydro-1,2,4-triazol-5-thiono-1-yl)-propan-2-ol in Form einer Festsubstanz, die bei 138 bis 139°C schmilzt.

## Patentansprüche

1. Verfahren zur Herstellung von Triazolinthion-Derivaten der Formel 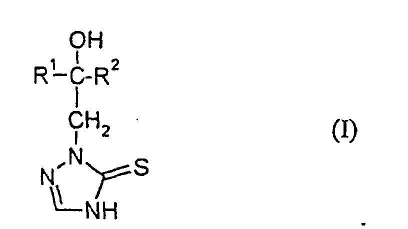 in welcher
R¹ für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und
R² für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
durch Reaktion von Triazol - Derivaten der Formel 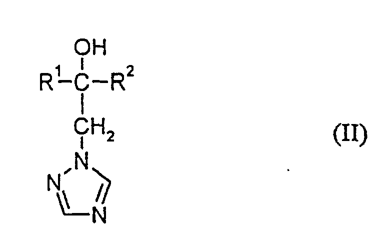 in welcher
R¹ und R² die oben angegebenen Bedeutungen haben,
mit Schwefel in Gegenwart eines aprotischen, polaren Verdünnungsmittels,
**dadurch gekennzeichnet, daß** man die Umsetzung bei Temperaturen zwischen 150 ° und 160 °C durchführt,
wobei die Mengen an den Reaktionskomponenten so gewählt werden, daß auf 1 Mol an Triazol-Derivat der Formel (II) zwischen 8 und 13 Mol Schwefel vorhanden sind,
und wobei während der Umsetzung Luft über das Reaktionsgemisch geleitet wird.

2. Verfahren gemäß Anspruch 1, **dadurch gekennzeichnet, daß** man als aprotisches, polares Verdünnungsmittel ein Amid, ein N-Alkyl-pyrrolidon oder ein N-Alkyl-caprolactam einsetzt.

3. Verfahren gemäß Anspruch 1, **dadurch gekennzeichnet, daß** man 2-(1-Chlorcyclopropyl)-1-(2-chlor-phenyl)-3-(1,2,4-triazol-1-yl)-propan-2-ol der Formel 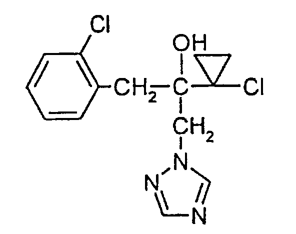 als Ausgangssubstanz einsetzt.

## Claims

1. Process for preparing triazolinethione derivatives of the formula 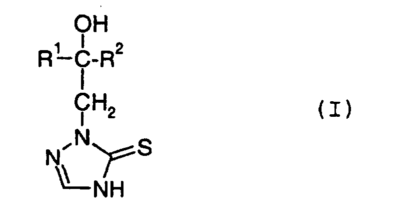 in which
R¹ represents phenylalkyl having 1 to 4 carbon atoms in the straight-chain or branched alkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenyl which may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
and
R² represents straight-chain or branched alkyl having 1 to 4 carbon atoms, where these radicals may be mono- to tetrasubstituted by identical or different substituents from the group consisting of fluorine, chlorine, bromine, methoxy, ethoxy, propoxy, isopropoxy, alkoximino having 1 or 2 carbon atoms in the alkoxy moiety, cyclopropyl, cyclobutyl, cyclopentyl and cyclohexyl,
or
represents cycloalkyl having 3 to 6 carbon atoms, where each of these radicals may be mono- to trisubstituted by identical or different substituents from the group consisting of fluorine, chlorine, bromine, cyano, methyl, ethyl, propyl, isopropyl and tert-butyl,
by reacting triazole derivatives of the formula 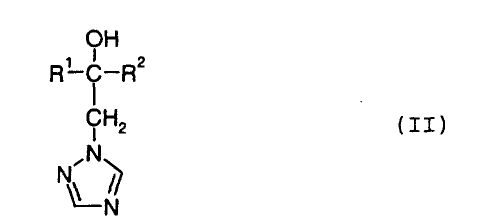 in which
R¹ and R² are each as defined above
with sulphur in the presence of an aprotic polar diluent, **characterized in that** the reaction is carried out at temperatures between 150°C and 160°C,
where the amounts of the reaction components are chosen such that between 8 and 13 mol of sulphur are present per mole of triazole derivative of the formula (II),
and where air is passed over the reaction mixture during the reaction.

2. Process according to Claim 1, **characterized in that** the aprotic polar diluent used is an amide, an N-alkyl-pyrrolidone or an N-alkyl-caprolactam.

3. Process according to Claim 1, **characterized in that** the starting material used is 2-(1-chloro-cyclopropyl)-1-(2-chloro-phenyl)-3-(1,2,4-triazol-1-yl)-propan-2-ol of the formula

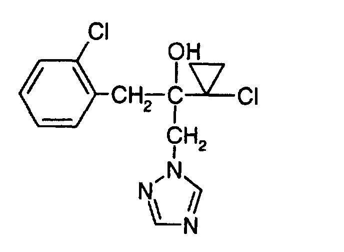

## Revendications

1. Procédé de production de dérivés de triazolinethione de formule 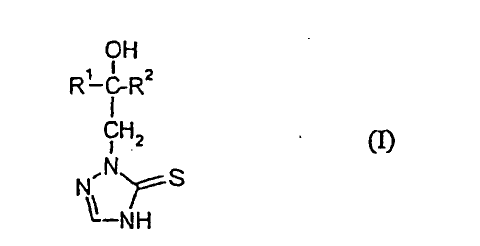 dans laquelle
R¹ est un reste phénylalkyle ayant 1 à 4 atomes de carbone dans la partie alkyle linéaire ou ramifiée, la partie phényle pouvant porter un à trois substituants, identiques ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou représente
un reste phényle, qui peut porter un à trois substituants, identiques ou différents, fluoro, chloro, bromo, méthyle, éthyle, tertiobutyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
et
R² représente un reste alkyle linéaire ou ramifié ayant 1 à 4 atomes de carbone, ces restes pouvant porter 1 à 4 substituants, identiques ou différents, fluoro, chloro, bromo, méthoxy, éthoxy, propoxy, isopropoxy, alkoximino ayant 1 ou 2 atomes de carbone dans la partie alkoxy, cyclopropyle, cyclobutyle, cyclopentyle et/ou cyclohexyle,
ou bien
un reste cycloalkyle ayant 3 à 6 atomes de carbone, chacun de ces restes pouvant porter un à trois substituants, identiques ou différents, fluoro, chloro, bromo, cyano, méthyle, éthyle, propyle, isopropyle et/ou tertiobutyle,
par réaction de dérivés triazoliques de formule 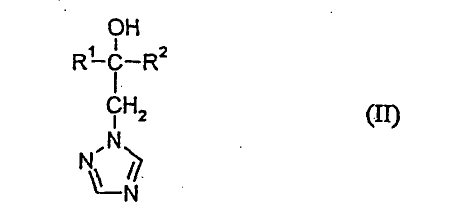 dans laquelle R¹ et R² ont les définitions indiquées ci-dessus,
avec le soufre en présence d'un diluent aprotique polaire,
**caractérisé en ce qu'**on conduit la réaction à des températures comprises entre 150° et 160°C,
les quantités des composants réactionnels étant choisies de manière qu'il y ait entre 8 et 13 moles de soufre par mole de dérivé triazolique de formule (II),
et de l'air étant envoyé sur le mélange réactionnel au cours de la réaction.

2. Procédé suivant la revendication 1, **caractérisé en ce qu'**on utilise comme diluent aprotique polaire un amide, une N-alkylpyrrolidone ou un N-alkylcaprolactame.

3. Procédé suivant la revendication 1, **caractérisé en ce qu'**on utilise comme composé de départ le 2-(1-chlorocyclopropyl)-1-(2-chlorophényl)-3-(1,2,4-triazole-1-yl)propane-2-ol de formule
